# FASCICULE DE BREVET EUROPEEN

(11) **EP 1 378 217 B1**
(45) Date de publication et mention de la délivrance du brevet: **09.04.2008**
(21) Numéro de dépôt: 03356100.2
(22) Date de dépôt: 01.07.2003
(51) Int. Cl.: A61F 5/058

(54) **Immobilisateur dynamique élastique pour doigts ou orteils**
Elastischer Halter für Finger oder Zehen
Elastical immobilisation device for fingers or toes

(30) Priorité: 02.07.2002 FR 0208255
(43) Date de publication de la demande: 07.01.2004
(73) Titulaire: Laboratoire Sober, 38920 Crolles (FR)
(72) Inventeur: Berrehail, Mohamed, 38240 Meylan (FR)
(74) Mandataire: Maureau, Philippe

(56) Documents cités:
- FR-A- 2 578 740
- SU-A- 1 378 827
- US-A- 4 615 046
- US-A- 5 267 945

## Description

La présente invention concerne un immobilisateur dynamique élastique pour doigts ou orteils, permettant de réaliser une syndactylie d'un ou plusieurs doigts.

Plus particulièrement, le dispositif selon la présente invention s'applique à la syndactylie des quatre derniers doigts de la main, ou des orteils.

Par "syndactylie", on entend le geste médical qui permet de lier temporairement et réversiblement plusieurs doigts ou orteils d'une main, au minimum un doigt malade avec un doigt sain, de sorte que le doigt sain serve de support au doigt malade.

De tels dispositifs de syndactylie sont utilisés de manière thérapeutique, pour réparer des traumatismes des doigts, des métacarpiens, des orteils ou des métatarsiens (fractures, luxations, entorses, plaies, brûlures, etc.). On réalise également des syndactylies en rhumatologie, dans le cas de certaines affections inflammatoires. On ne réalise en principe pas de syndactylies du pouce, étant donné que c'est un doigt opposable, et compte tenu de sa géométrie par rapport aux autres doigts, il est assez difficilement syndactylisable à l'index.

Habituellement, les dispositifs médicaux utilisés pour réaliser une syndactylie sont constitués d'une attelle en plastique rigide ou en métal recouverte de tissu, ou de dispositifs semi-rigides en tissu adhésif de type Elastoplast^{™} ou non adhésif auto-agrippant de type Velcro®.

Un tel dispositif est décrit par exemple dans la demande de brevet FR 2578740.

Un désavantage majeur de tels dispositifs réside dans ce qu'ils n'ont aucune fonction dynamique de rééducation précoce pendant la période de guérison. En particulier, ils n'autorisent pas le coulissement d'un doigt par rapport aux autres doigts syndactylisés.

Un objectif de la présente invention est donc de fournir un dispositif permettant de réaliser la syndactylie de deux ou plusieurs doigts ou orteils, qui soit économique à fabriquer, simple à utiliser, et qui permette la rééducation dynamique précoce du doigt malade.

Le but précité est atteint avec un dispositif médical pour la réalisation d'une syndactylie d'au moins deux doigts ou orteils, caractérisé en ce qu'il est monopièce et comprend au moins deux anneaux souples reliés par au moins une barrette de liaison souple, de manière à permettre le coulissement des doigts ou orteils syndactylisés, les uns par rapport aux autres lors des mouvements de flexion/extension.

Selon un mode de réalisation de l'invention, le dispositif de syndactylie comprend deux barrettes de liaison croisées, disposées entre au moins deux anneaux. Selon un mode de réalisation alternatif de l'invention, il comprend au moins deux barrettes de liaison parallèles les unes aux autres, disposées entre au moins deux anneaux.

De préférence, chaque barrette du dispositif est une barrette dorsale et/ou latérale, et également de préférence les anneaux du dispositif peuvent être solidaires d'un second dispositif.

Avantageusement, le dispositif selon l'invention est réalisé en un seul et même matériau élastomère à propriétés résilientes. Ceci permet de réduire les coûts de fabrication, et donc de fournir un dispositif efficace à moindre coût pour le patient.

Selon un mode de réalisation de l'invention, le dispositif de syndactylie est réalisé en deux matériaux, l'un souple pour les anneaux et l'autre plus rigide pour la ou les barrette(s).

De toute façon, l'invention sera bien comprise, à l'aide de la description qui suit, en référence au dessin schématique annexé représentant, à titre d'exemples non limitatifs, plusieurs formes préférées du dispositif de syndactylie selon l'invention.

Figure 1 est une vue de dessus en perspective d'un premier dispositif à barrette simple.

Figure 2 est une vue de dessus en perspective du dispositif de figure 1, mis en place sur deux doigts d'une main d'un patient.

Figure 3 est une vue de dessus en perspective d'un dispositif à barrette double croisée selon l'invention, mis en place sur la main d'un patient.

Figure 4 est une vue de dessus en perspective d'un second dispositif selon l'invention avec une barrette supérieure et deux barrettes latérales, mis en place sur la main d'un patient.

Figure 5 est une vue de dessus en perspective de deux dispositifs selon l'invention jumelés et mis en place autour des doigts d'un patient.

Figure 6 est une vue en perspective d'un dispositif selon figure 1, lorsque les doigts sont en flexion.

Le dispositif 1 permettant la syndactylie d'au moins deux doigts ou orteils selon l'invention, comprend au moins deux anneaux 2 souples reliés par au moins une barrette de liaison 3 souple, de manière à permettre le coulissement des doigts ou orteils syndactylisés 4, les uns par rapport aux autres. Dans la description qui suit, on fera référence à la syndactylie de doigts, mais il ne s'agit en aucun cas d'une description restrictive de l'invention, cette dernière étant prévue pour réaliser de préférence une syndactylie parmi les quatre premiers doigts de la main, ainsi que parmi les cinq orteils.

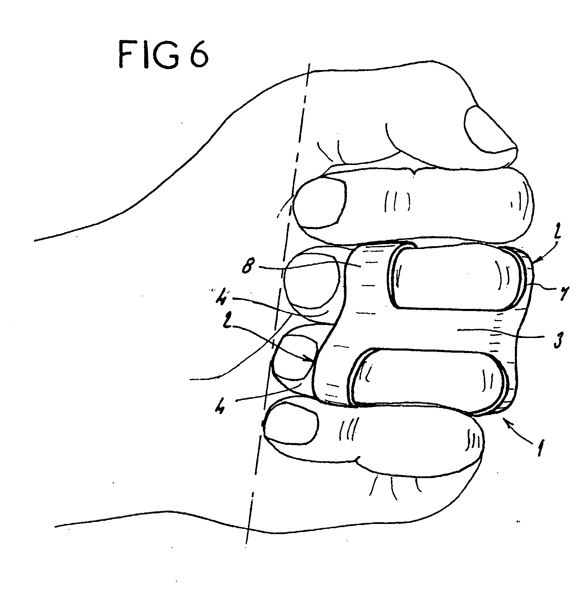

Selon une caractéristique essentielle de l'invention, le dispositif 1 permet le coulissement des doigts syndactylisés 4 les uns par rapport aux autres, lors du mouvement de flexion/extension desdits doigts. Cette indépendance des doigts syndactylisés 4 en terme de coulissement, permet de conserver une mobilité réduite, mais essentielle du doigt malade, au moins dans un plan. Ainsi, la guérison est possible grâce au maintien, principalement latéral du doigt malade par le ou les doigt(s) adjacent(s), et simultanément, la rééducation précoce est possible puisque la motricité du doigt malade est préservée, au moins pour ce qui est des mouvements de flexion/extension. La guérison sans altération de la motricité est ainsi possible. Un tel coulissement des doigts les uns par rapport aux autres est réalisé lorsqu'en flexion complète de la main, les extrémités des quatre derniers doigts sont alignés, comme cela est montré à la figure 6, ce qui n'est pas le cas lorsque la main est en extension, même partielle.

Ce dispositif 1 est de préférence moulé à plat en une seule pièce, de préférence par injection d'un élastomère thermoplastique à propriétés résilientes. De telles propriétés de résilience permettent de réaliser une syndactylie de qualité, c'est-à-dire une liaison efficace d'un doigt malade avec un ou plusieurs doigts adjacents, tout en gardant une souplesse et une capacité d'étirement qui permet une mise en place et un retrait faciles. En outre, l'élasticité des anneaux du dispositif selon l'invention permet aux doigts syndactylisés 4 de coulisser les uns par rapport aux autres, ce qui est, comme déjà expliqué ci-avant, une composante essentielle du dispositif de l'invention.

Eventuellement, on peut envisager que le dispositif de syndactylie 1 de l'invention est réalisé par bi-injection, c'est-à-dire injection séquentielle dans le même moule de deux matières plastiques différentes. Ainsi, les anneaux 2 peuvent être moulés dans un premier matériau élastique afin de permettre la mise en place et le retrait autour des doigts de même qu'un bon maintien lorsque l'anneau est positionné autour des doigts, et la barrette 3 qui les relie peut être moulée dans un matériau souple mais non élastique, de manière à conserver toujours constante la distance entre les deux anneaux 2 du dispositif 1.

Dans tous les cas, le matériau choisi pour réaliser le dispositif de l'invention est de préférence un matériau non allergisant.

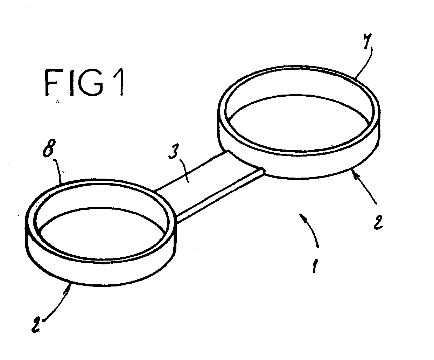

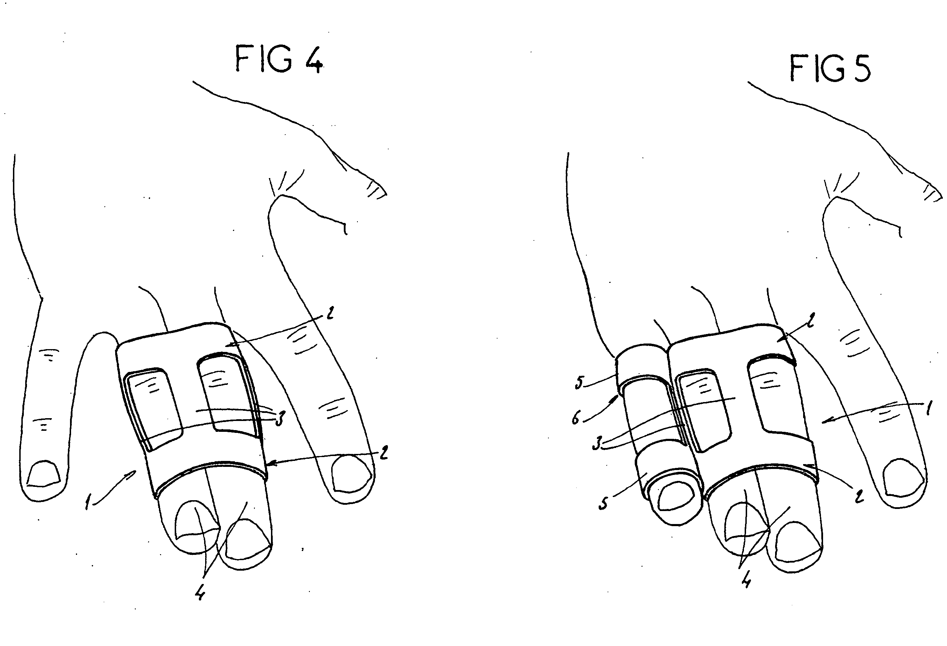

Selon un premier mode de réalisation de l'invention, tel que représenté aux figures 1 et 5, le dispositif de syndactylie 1 selon l'invention comprend une unique barrette de liaison 3 disposée entre au moins deux anneaux 2.

Selon un second mode de réalisation de l'invention, tel que représenté à la figure 4, le dispositif de syndactylie 1 selon l'invention comprend deux barrettes de liaison 3 croisées, disposées entre au moins deux anneaux 2.

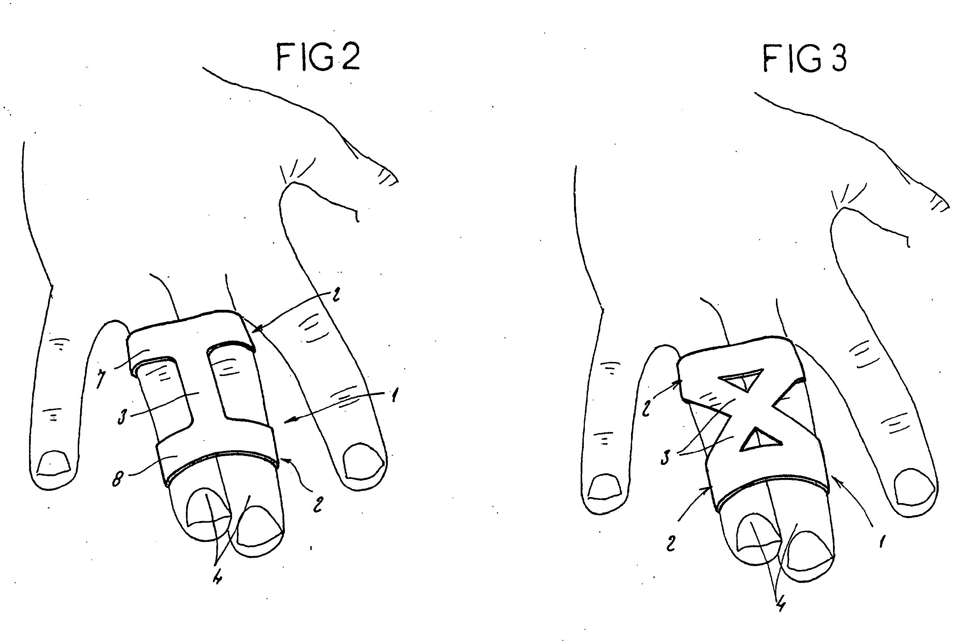

Selon un troisième mode de réalisation de l'invention, représenté à la figure 2, le dispositif 1 comprend au moins deux barrettes de liaison 3 parallèles les unes aux autres, disposées entre au moins deux anneaux 2.

Dans chacun des différents modes de réalisation de l'invention décrits ci-dessus, chaque barrette 3 du dispositif 1 est une barrette dorsale et/ou latérale, c'est-à-dire que la ou les barrettes) est/sont destinée(s) à être disposée(s) du côté dorsal de la main ou du pied, et/ou éventuellement sur le côté latéral du groupe de doigts syndactylisés. En effet, dans le cas où une barrette serait placée sur la face palmaire de la main ou du pied, celle-ci serait sujet à des plis très inconfortables pour le patient, lors des mouvements de flexion.

Dans un mode particulier de réalisation du dispositif selon l'invention, tel que représenté à la figure 4, les anneaux 2 du dispositif 1 peuvent être solidaires des anneaux 5 d'un second dispositif de syndactylie 6, ou alternativement, ils peuvent être solidaires d'anneaux 5 passés autour d'un doigt sain.

Comme on le voit sur les figures 2 à 5, la réalisation d'une syndactylie à l'aide du dispositif selon l'invention s'opère en enfilant l'un après l'autre les anneaux 2 du dispositif 1 simultanément autour des doigts 4 à syndactyliser. La distance entre les anneaux 2 est déterminée par la longueur de la barrette de liaison 3. Le diamètre des anneaux 2 est calculé pour correspondre environ à la somme des diamètres des doigts 4 à syndactyliser. En tout état de cause, l'homme du métier saura adapter ce diamètre en fonction de la taille des doigts du patient, ainsi que la largeur des anneaux 2, de sorte que la liaison entre les deux doigts adjacents soit réalisée sans jeu (c'est-à-dire que l'anneau soit légèrement sous tension une fois celui-ci mis en place autour des doigts), tout en permettant une circulation sanguine correcte dans les doigts, et en autorisant le coulissement des doigts les uns par rapport aux autres. De manière similaire, l'épaisseur des anneaux pourra être adaptée selon la force élastique désirée, en fonction de l'indication thérapeutique.

De même la longueur de la barrette de liaison 3 devra être choisie pour permettre l'adaptation du dispositif de syndactylie 1 selon l'invention de la manière suivante: l'anneau de plus grand diamètre 7 étant passé le premier et disposé autour des phalanges proximales des doigts à syndactyliser 4, puis l'anneau de plus faible diamètre 8 étant passé autour des phalanges distales des doigts à syndactyliser 4, de sorte que la (ou les) barrette(s) de liaison 3 soi(en)t disposée(s) au-dessus de l'articulation entre lesdites phalanges proximales et distales. De la sorte, on peut librement choisir de disposer le dispositif autour de la première ou de la seconde articulation interphalangienne du doigt malade.

Enfin, il est à noter que le dispositif selon l'invention est prévu pour être utilisé lorsque au moins certains doigts du patient sont recouverts de pansements, par exemple dans le cas de brûlures. Dans ce cas toutefois, il faudra veiller à adapter le diamètre des anneaux du dispositif.

Tout en ayant essayé, dans la spécification qui précède, d'attirer l'attention sur les caractéristiques de l'invention jugées revêtir une importance particulière, il faut noter que l'invention ne se limite pas au mode de réalisation décrit ci-avant, mais qu'elle en embrasse au contraire toutes les variantes. C'est ainsi, notamment que le nombre et la disposition des barrettes de liaison peuvent être adaptés en fonction de la morphologie du patient, ou en fonction de la rééducation à effectuer.

## Revendications

1. - Dispositif médical (1) pour la réalisation d'une syndactylie d'au moins deux doigts ou orteils (4), **caractérisé en ce qu**'il est monopièce et comprend au moins deux anneaux (2) souples, reliés par au moins une barrette de liaison (3) souple, de manière à permettre le coulissement des doigts ou orteils syndactylisés (4), les uns par rapport aux autres.

2. - Dispositif de syndactylie (1) selon la revendication 1, **caractérisé en ce qu**'il comprend deux barrettes de liaison (3) croisées, disposées entre au moins deux anneaux (2).

3. - Dispositif de syndactylie (1) selon la revendication 1, **caractérisé en ce qu**'il comprend au moins deux barrettes de liaison (3) parallèles les unes aux autres, disposées entre au moins deux anneaux (2).

4. - Dispositif de syndactylie (1) selon l'une quelconque des revendications 1 à 3, **caractérisé en ce que** chaque barrette (3) du dispositif (1) est une barrette dorsale et/ou latérale.

5. - Dispositif de syndactylie (1) selon l'une quelconque des revendications 1 à 4, **caractérisé en ce que** les anneaux (2) du dispositif (1) sont solidaires d'un second dispositif de syndactylie (6).

6. - Dispositif de syndactylie (1) selon l'une quelconque des revendications 1 à 5, **caractérisé en ce qu'**il est réalisé en un seul et même matériau élastomère à propriétés résilientes.

7. - Dispositif de syndactylie (1) selon l'une quelconque des revendications 1 à 5, **caractérisé en ce qu**'il est réalisé en deux matériaux, l'un souple pour les anneaux (2), et l'autre plus rigide pour la ou les barrette(s) de liaison (3).

## Claims

1. Medical device (1) for syndactyly of at least two fingers or toes (4), **characterized in that** it is in one piece and comprises at least two flexible rings (2) joined via at least one flexible connection bar (3) in such a way as to permit sliding of the syndactylized fingers or toes (4) relative to one another.

2. Syndactyly device (1) according to Claim 1, **characterized in that** it comprises two intersecting connection bars (3) arranged between at least two rings (2).

3. Syndactyly device (1) according to Claim 1, **characterized in that** it comprises at least two connection bars (3) parallel to one another and arranged between at least two rings (2).

4. Syndactyly device (1) according to any one of Claims 1 to 3, **characterized in that** each bar (3) cf the device (1) is a dorsal and/or lateral bar.

5. Syndactyly device (1) according to any one of Claims 1 to 4, **characterized in that** the rings (2) of the device (1) are integral with a second syndactyly device (6).

6. Syndactyly device (1) according to any one of Claims 1 to 5, **characterized in that** it is made of a single elastomeric material with resilient properties.

7. Syndactyly device (1) according to any one of Claims 1 to 5, **characterized in that** it is made of two materials, one flexible for the rings (2), and the other more rigid for the connection bar or bars (3).

## Patentansprüche

1. Medizinische Vorrichtung (1) für die Syndaktylisierung von mindestens zwei Fingern oder Zehen (4), **dadurch gekennzeichnet, dass** sie einstückig ist und mindestens zwei flexible Ringe (2) aufweist, die über mindestens einen flexiblen Verbincungssteg (3) so verbunden sind, dass die syndaktylisierten Finger oder Zehen (4) bezüglich einander gleiten können.

2. Syndaktylisierungsvorrichtung (1) nach Anspruch 1, **dadurch gekennzeichnet, dass** sie zwei überkreuzte verbindungsstege (3) aufweist, die zwischen mindestens zwei Ringen (2) angeordnet sind.

3. Syndaktylisierungsvorrichtung (1) nach Anspruch 1, **dadurch gekennzeichnet, dass** sie mindestens zwei parallel zueinander verlaufende Verbindungsstege (3) aufweist, die zwischen mindestens zwei Ringen (2) angeordnet sind.

4. Syndaktylisierungsvorrichtung (1) nach einem der Ansprüche 1 bis 3, **dadurch gekennzeichnet, dass** es sich bei jedem Steg (3) der Vorrichtung (1) um einen dorsalen und/oder lateralen Steg handelt.

5. Syndaktylisierungsvorrichtung (1) nach einem der Ansprüche 1 bis 4, **dadurch gekennzeichnet, dass** eie Ringe (2) der Vorrichtung (1) fest mit einer zweiten Syndaktylisierungsvorrichtung (6) verbunden sind.

6. Syndaktylisicrungsvorrichtung (1) nach einem der Ansprüche 1 bis 5, **dadurch gekennzeichnet, dass** sie aus einem einzigen elastomeren Material mit elastischen Eigenschaften hergestellt ist.

7. Syndaktylisierungsvorrichtung (1) nach einem der Ansprüche 1 bis 5, **dadurch gekennzeichnet, dass** sie aus zwei Materialien hergestellt ist, das eine flexibel für die Ringe (2) und das andere starrer für den oder die verbindungssteg(e) (3).
